# EUROPEAN PATENT APPLICATION

(11) **EP 3 922 297 A2**
(43) Date of publication of application: **15.12.2021**
(21) Application number: 20795823.2
(22) Date of filing: 24.04.2020
(51) Int. Cl.: A61M 37/00, A61M 35/00

(54) **DEVICE FOR SKIN CARE, COSMETICS, AND MEDICAL TREATMENT**

(30) Priority: 26.04.2019 KR 20190001734 U
(71) Applicant: DSTECH Co., Ltd., Okcheon-gun, Chungcheongbuk-do 29055 (KR)
(72) Inventor: JANG, Min Seo, Seoul 08101 (KR)
(74) Representative: Isarpatent
(86) International application number: PCT/KR2020/005406
(87) International publication number: WO 2020/218862

(57) **Abstract**

The present invention provides a device for skin care, cosmetics and medical treatment, which includes a roller coupled with microneedles and a handle unit for movement coupled with the roller to move the roller. The handle unit for movement may include a roller coupling part to which the roller is detachably coupled, an elastic pressing part connected to the roller coupling part to adjust a width thereof, and a grip part extended from the elastic pressing part. Accordingly, the roller may be easily attached and detached.

## Description

### [Technical Field]

The present invention relates to a device for skin care, cosmetics and medical treatment, and more specifically to a device for skin care, cosmetics and medical treatment including a roller.

### [Background Art]

Recently, as a method for efficiently delivering skin care substances to an epidermal layer or a dermis layer without stimulating pain spots in the skin, microneedles are attracting attention. In particular, the microneedles are manufactured and have become commercially available in a form of patches or rollers.

For example, Korean Patent Laid-Open Publication No. 10-2015-0028248 discloses a patch-type product in which microneedles are processed with biodegradable cosmetic materials themselves.

However, the patch-type microneedles have limitations in producing products having various shapes and sizes required according to curves and a size of the face. In addition, there are problems in that it is difficult to produce the patch-type product in large quantities or high costs are required to manufacture a mold.

Therefore, there is a need to develop a microneedle product having a shape capable of being used without limitation in the curves and size of the face.

### [Summary of Invention]

### [Problems to be Solved by Invention]

It is an object of the present invention to provide a device for skin care, cosmetics and medical treatment with improved ease of use.

### [Means for Solving Problems]

To achieve the above objects, the following technical solutions are adopted in the present invention.
1. A device for skin care, cosmetics and medical treatment including: a roller to which microneedles are coupled; and a handle unit for movement coupled to the roller to move the roller, wherein the handle unit for movement includes: a roller coupling part to which the roller is detachably coupled; an elastic pressing part connected to the roller coupling part to adjust a width thereof; and a grip part extended from the elastic pressing part.
2. The device for skin care, cosmetics and medical treatment according to the above 1, wherein the roller is coupled with a microneedle sheet having the microneedles formed thereon.
3. The device for skin care, cosmetics and medical treatment according to the above 1, further including a cushioning cylinder mounted on the roller to cushion an impact applied to the roller.
4. The device for skin care, cosmetics and medical treatment according to the above 3, wherein the microneedles are coupled to the cushioning cylinder mounted on the roller.
5. The device for skin care, cosmetics and medical treatment according to the above 1, wherein the roller coupling part is deformed so that the roller is attached thereto or detached therefrom by a tension transmitted from the elastic pressing part due to a pressure applied thereto and converted into a tensile force.
6. The device for skin care, cosmetics and medical treatment according to the above 5, wherein the roller coupling part includes a pair of roller support parts facing each other with the roller interposed therebetween.
7. The device for skin care, cosmetics and medical treatment according to the above 6, wherein a width between the roller support parts is changed by the tension.
8. The device for skin care, cosmetics and medical treatment according to the above 6, wherein the roller includes rotation axis parts, and the roller support parts includes roller connection parts coupled with the rotation axis parts.
9. The device for skin care, cosmetics and medical treatment according to the above 6, wherein regions between the pair of roller support parts comprise: a coupling region into which the roller is inserted; and a first space excluding the coupling region among the regions between the roller support parts.
10. The device for skin care, cosmetics and medical treatment according to the above 1, wherein the elastic pressing part includes: an elastic lever to which a pressure is applied; and a lever support part facing the elastic lever with being spaced apart therefrom.
11. The device for skin care, cosmetics and medical treatment according to the above 10, wherein a second space is formed between the elastic lever and the lever support part.
12. The device for skin care, cosmetics and medical treatment according to the above 10, wherein the grip part is extended from the lever support part.

### [Advantageous Effects]

The device for skin care, cosmetics and medical treatment according to embodiments of the present invention allows a user to easily couple or separate the roller by adjusting the width of the roller coupling part. Therefore, the user may easily change types of the roller as necessary.

In addition, it is possible to effectively prevent a problem in which the roller is contaminated during replacing the rollers.

### [Brief Description of Drawings]

FIG. 1 is a schematic perspective view illustrating a device for skin care, cosmetics and medical treatment according to exemplary embodiments.
FIG. 2 is a schematic cross-sectional view illustrating a roller according to some exemplary embodiments.
FIG. 3 and 4 are schematic perspective views illustrating rollers according to some exemplary embodiments.
FIG. 5 is a schematic plane view illustrating a roller coupling part according to some exemplary embodiments.
FIG. 6 is a schematic plane view illustrating a method for adjusting a width of the roller coupling part according to some exemplary embodiments.
FIG. 7 is a schematic view for describing a method for coupling the roller according to some exemplary embodiments.

### [Mode for Carrying out Invention]

The device for skin care, cosmetics and medical treatment according to embodiments of the present invention allows a user to adjust the width of the roller coupling part from which the roller coupled with the microneedle is detached, so that the roller may be easily coupled to the device for skin care, cosmetics and medical treatment or may be separated therefrom.

Hereinafter, with reference to the drawings, embodiments of the present invention will be described in more detail. However, since the drawings attached to the present disclosure are only given for illustrating one of preferable various embodiments of present invention to easily understand the technical idea of the present invention with the above-described invention, it should not be construed as limited to such a description illustrated in the drawings.

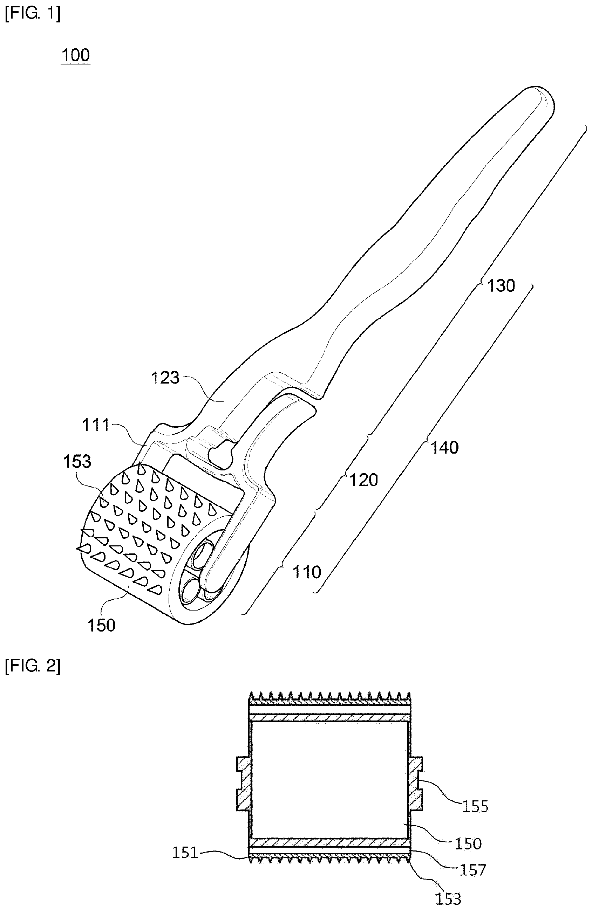

FIG. 1 is a schematic perspective view illustrating a device for skin care, cosmetics and medical treatment according to exemplary embodiments.

Referring to FIG. 1, a device 100 for skin care, cosmetics and medical treatment may include a roller 150 to which microneedles 153 are coupled, and a handle unit 140 for movement coupled to the roller 150 to move the roller 150.

For example, the roller 150 is coupled with the microneedles 153, and may serve to support the microneedles 153 to be inserted into the skin.

For example, the roller 150 may have shapes such as a cylindrical shape, a triangular prism shape, a square prism shape, or a polygonal prism shape.

For example, the microneedles 153 may have a needle shape protruding from a surface of the roller 150.

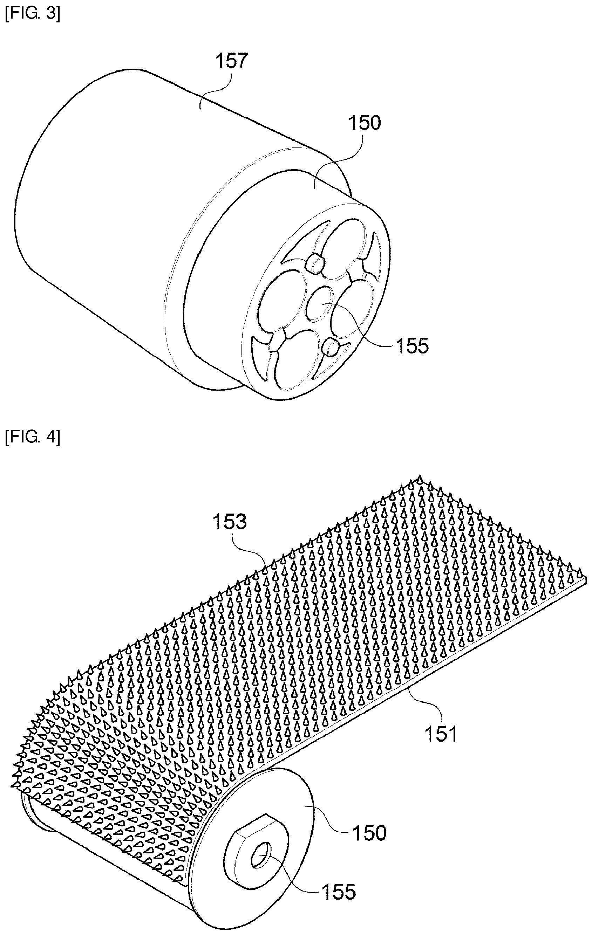

FIG. 2 is a schematic cross-sectional view illustrating a roller according to some exemplary embodiments. FIG. 3 and 4 are schematic perspective views illustrating rollers according to some exemplary embodiments.

Referring to FIG. 2, the roller 150 may include a pair of rotation axis parts 155 provided on both sides of the roller 150 and coupled to one end of the handle unit 140 for movement.

In some exemplary embodiments, a cushioning cylinder 157 may be mounted on the roller 150 to cushion an impact applied to the roller 150.

Referring to FIG. 3, for example, the roller 150 may be inserted into the cylinder-shaped cushioning cylinder 157 to be coupled thereto. For example, a band-shaped cushioning cylinder 157 may be wound on a circumferential surface of the roller 150 to be mounted thereon.

A material of the cushioning cylinder 157 is not particularly limited so long as it is a material that can mitigate the impact applied to the roller 150. For example, the material of the cushioning cylinder 157 may include silicone or urethane, etc.

In some exemplary embodiments, the microneedles 153 may be coupled to the cushioning cylinder 157 mounted on the roller 150. For example, a microneedle sheet 151 having the microneedles 153 formed thereon may be coupled to the cushioning cylinder 157.

In one embodiment, the microneedle sheet 151 may be wound on the circumferential surface of the cushioning cylinder 157 to be coupled thereto.

In this case, adhesion between the skin and the roller 150 is further improved by the cushioning cylinder 157, such that the microneedles 153 may be more easily inserted into the skin.

In some exemplary embodiments, the roller 150 may be coupled with the microneedle sheet 151 on which the microneedles 153 are formed.

Referring to FIG. 4, the microneedle sheet 151 having the microneedles 153 formed thereon may be directly coupled to the circumferential surface of the roller 150.

For example, the microneedle sheet 151 may be wound on the roller 150 to be coupled thereto. For example, the roller 150 may be inserted into the cylindrical microneedle sheet 151 to be coupled thereto.

In exemplary embodiments, the microneedle sheet 151 may be separated from the roller 150 or the cushioning cylinder 157.

Accordingly, the microneedle sheet 151 including a damaged microneedle 153 may be removed, and then replaced with a new microneedle sheet 151. In this case, a service life of the device 100 for skin care, cosmetics and medical treatment may be increased.

For example, the microneedle sheet 151 may be coupled to the circumferential surface of the roller 150 or the cushioning cylinder 157 to form a single body with the roller 150 or the cushioning cylinder 157. For example, the microneedles 153 may be directly formed on the circumferential surface of the roller 150 or the cushioning cylinder 157.

For example, a height at which the microneedle 153 protrudes from the circumferential surface of the roller 150 or the cushioning cylinder 157 may be about 100 µm to 850 µm. For example, the height at which the microneedle 153 protrudes from a surface of the microneedle sheet 151 may be about 100 µm to 850 µm. Within the above range, the microneedles 153 may be easily inserted under a horny layer of the skin.

In some embodiments, the microneedles 153 may include a biodegradable material. For example, the microneedle 153 may include hyaluronic acid, gelatin or glycerin, etc.

In some embodiments, the microneedles 153 may include drugs (not illustrated). For example, the drug may be located inside the microneedle 153. Alternately, the drug may be coated on the surface of the microneedle 153.

For example, the drug collectively refers to a substance or compound which is absorbed into the skin to generate a skin beautifying effect, a skin treatment effect, and a skin improvement effect. Meanwhile, the drug may include a skin beautifying agent, a medicinal preparation, or a skin improving agent.

For example, the skin care agent may be at least one selected from the group consisting of a skin moisturizing agent, a skin whitening agent, a wrinkle improving agent, an acne improving agent, a melasma/freckle improving agent, an anti-hair loss agent, a hair growth promoting agent, and an anti-aging agent.

For example, the medicinal preparation may be at least one selected from the group consisting of analgesic, therapeutic agent for arthritis, therapeutic agent for osteoporosis, insulin, vaccine, nicotine, and therapeutic agent for immune skin diseases.

For example, the skin improving agent may be at least one selected from the group consisting of Botox, fillers, local lipolytic agent, therapeutic agent for skin inflammation, corn remover, wart remover and the like.

Referring to FIG. 1, the roller 150 may be coupled to the handle unit 140 for movement. For example, the roller 150 may be moved by the handle unit 140 for movement.

In one embodiment, the roller 150 may be moved while rotating about an axis passing through the pair of rotation axis parts 155.

The handle unit 140 for movement may support the roller 150 so that the microneedles 153 coupled thereto are easily inserted into the skin while moving the roller 150. Accordingly, an absorption rate of drugs or cosmetics applied to the skin may be further improved.

The material of the handle unit 140 for movement is not particularly limited, and may include, for example, metal, plastic, wood, ceramics, or carbon fiber, etc.

Referring to FIG. 1, the handle unit 140 for movement may include a roller coupling part 110 to which the roller 150 is detachably coupled, an elastic pressing part 120 connected to the roller coupling part 110 to adjust a width thereof, and a grip part 130 extended from the elastic pressing part 120.

In some embodiments, the roller coupling part 110 may be deformed so that the roller 150 can be attached thereto or detached therefrom by a tension transmitted from the elastic pressing part 120 due to a pressure applied thereto and converted into a tensile force.

For example, the pressure applied to the elastic pressing part 120 may be transmitted to the roller coupling part 110 to change the width of the roller coupling part 110 in a lateral direction.

For example, the tension may mean a force applied in a direction opposite to a direction in which the pressure is applied to the elastic pressing part 120. For example, the tension may mean a force applied in a direction in which the width of the roller coupling part 110 is increased.

For example, the width of the roller coupling part 110 may mean a width of a coupling region into which the roller 150 is inserted and coupled. For example, the width of the roller coupling part 110 may be greater than or equal to the width of the roller 150.

For example, when the pressure is applied to the elastic pressing part 120, the width of the roller coupling part 110 may be increased by the tension, such that the roller 150 may be separated from the roller coupling part 110.

For example, when the applied pressure is removed to eliminate the tension, the width of the roller coupling part 110 may be narrowed, and the roller 150 may be maintained with being coupled to the roller coupling part 110.

Accordingly, the user may easily couple or separate the roller 150 to or from the roller coupling part 110 using the lever principle.

In addition, since there is no need to directly apply a force to the roller 150 when coupling or separating the roller 150, it is possible to prevent a problem in which the roller 150 is contaminated during coupling or separating the roller 150.

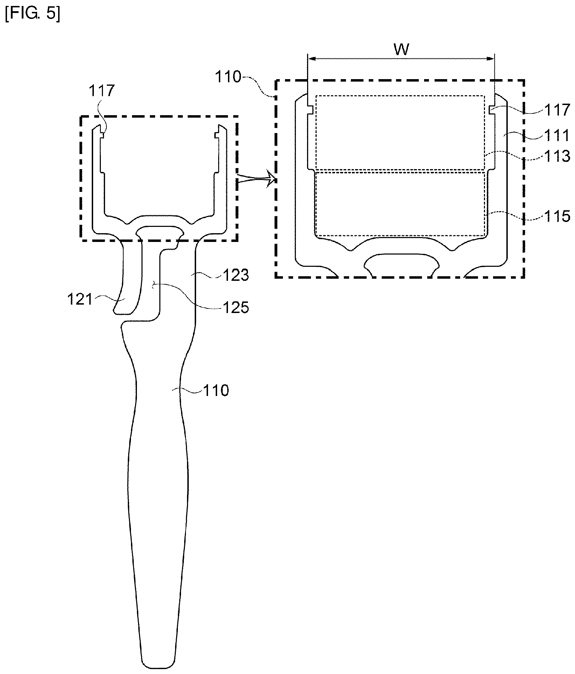

FIG. 5 is a schematic plane view illustrating the roller coupling part 110 according to some exemplary embodiments.

Referring to FIGS. 1 and 5, the roller coupling part 110 may include a pair of roller support parts 111 facing each other with the roller 150 interposed therebetween.

For example, the roller 150 may be coupled to the roller coupling part 110 through the roller support parts 111. In this case, the roller support parts 111 may prevent the roller 150 from being moved left and right in a moving direction in the process of moving the roller 150. Accordingly, the user may easily move the roller 150 in a predetermined direction on the skin.

In some exemplary embodiments, the width between the roller support parts 111 may be changed by the tension.

For example, the width between the roller support parts 111 may be changed by the tension. For example, the width between the roller support parts 111 may be changed so as to be greater than or equal to the width of the roller 150 by the tension.

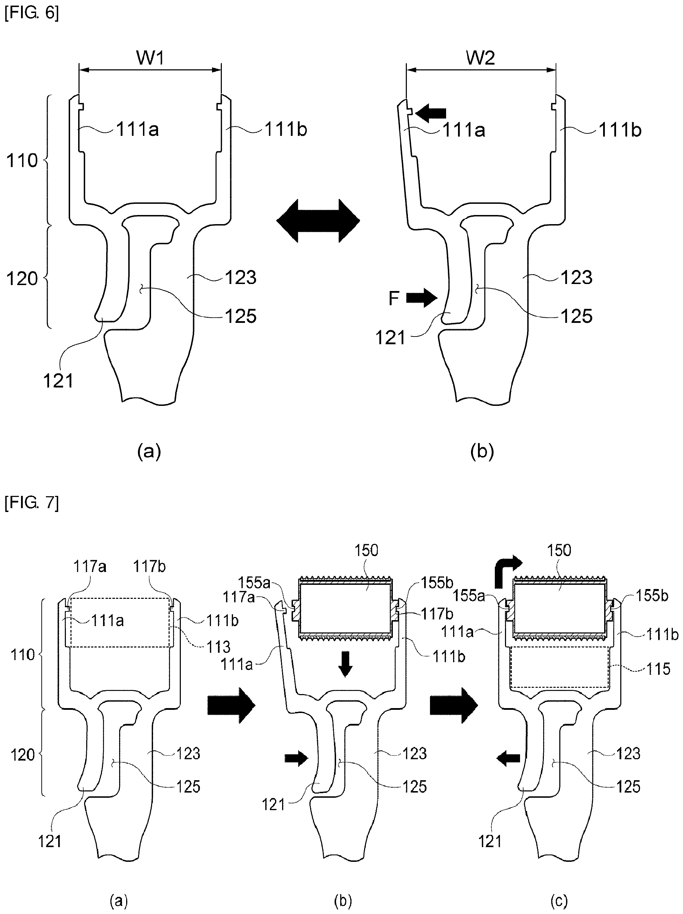

FIG. 6 is a schematic plane view illustrating a method for adjusting a width of the roller coupling part according to some exemplary embodiments.

Referring to FIG. 6, the width between the roller support parts 111a and 111b may be increased (e.g., from a width W1 to a width W2) by the tension transmitted to the roller support part 111a. In this case, the width W2 between the roller support parts 111a and 111b may be greater than the width of the roller 150.

For example, when the tension transmitted to the roller support part 111a is removed, the width between the roller support parts 111a and 111b may be decreased (e.g., from the width W2 to the width W1). In this case, the width W1 between the roller support parts 111a and 111b may be equal to the width of the roller 150.

For example, when the width between the roller support parts 111a and 111b is increased by the tension, the roller 150 may be separated from the roller coupling part 110, and when the width between the roller support parts 111a and 111b is decreased by removing the tension, the roller 150 may be maintained with being coupled to the roller coupling part 110.

In some exemplary embodiments, the roller 150 may include the rotation axis parts 155, and the roller support parts 111 may include roller connection parts 117 to be inserted into the rotation axis parts 155.

Referring to FIGS. 2 and 5, for example, the rotation axis parts 155 may have a shape recessed inward from both sides of the roller 150, and the roller connection parts 117 may have a shape protruding from the roller support parts 111 corresponding thereto. In this case, the roller connection parts 117 may be inserted into the rotation axis parts 155 to be coupled thereto.

In another embodiment, the rotation axis parts 155 may have a shape protruding from the both sides of the roller 150, and the roller connection parts 117 may have a shape recessed into the roller support parts 111 corresponding thereto. In this case, the rotation axis parts 155 may be inserted into the roller connection parts 117 to be coupled thereto.

Further, in another embodiment, the rotation axis parts 155 may have a shape recessed into the roller 150, and the roller connection parts 117 may also have a shape recessed from the surfaces of the roller support parts 111. In this case, rod shafts (not illustrated) may be inserted into the rotation axis parts 155 and the roller connection parts 117, respectively, to couple the roller 150 to the roller coupling part 110.

In some embodiments, referring to FIG. 5, regions between the pair of roller support parts 111 may include a coupling region 113 into which the roller 150 is inserted and a first space region 115 excluding the coupling region 113 among the regions between the roller support parts 111.

For example, the roller 150 may be inserted into the coupling region 113, and the roller 150 may be coupled to the pair of roller support parts 111.

For example, the first space region 115 may mean the remaining region where the roller 150 is not located among the regions between the pair of roller support parts 111. In other words, the first space region 115 may mean a region between the roller 150 and the elastic pressing part 120.

In this case, a sufficient distance may be secured between the elastic pressing part 120 and the roller 150 by the first space region 115. Thereby, a rate of change of the width between the roller support parts 111 may be increased.

For example, the rate of change of the width may mean a ratio of a width distance between the roller support parts 111 after application of the pressure to a width distance between the roller support parts 111 before application of the pressure.

In some exemplary embodiments, the rate of change of the width may be 1.1 to 1.5. Within the above range, the roller 150 may be more easily coupled to or separated from the roller coupling part 110.

In some exemplary embodiments, a ratio of a length of the first space region 115 to a length of the coupling region 113 may be 2 to 4. Since the rate of change of the width is appropriate within the above range, the roller 150 may be more easily coupled to or separated from the roller coupling part 110.

In addition, when satisfying the above-described length ratio, a supporting force of the handle unit 140 for movement with respect to the roller 150 may be excellent.

In some exemplary embodiments, referring to FIG. 6(a), the elastic pressing part 120 may include an elastic lever 121 to which a pressure F such as an external force is applied, and a lever support part 123 facing the elastic lever 121 with being spaced apart therefrom.

For example, the elastic lever 121 may be extended from the roller support part 111 in a longitudinal direction.

For example, the pressure F may be applied to the elastic lever 121. For example, the pressure F applied to the elastic lever 121 may be converted into a tensile force, such that tension due to the tensile force may be applied to the roller support part 111.

For example, the lever support part 123 may support the elastic lever 121, which allows the user to apply the pressure F to the elastic lever 121.

For example, the user may apply the pressure to the elastic lever 121 by applying a lateral pressure to the elastic lever 121 and the lever support part 123 at the same time, while grasping the elastic lever 121 and the lever support part 123.

For example, the lateral pressure may mean a force applied in a direction in which the elastic lever 121 and the lever support part 123 approach each other.

In this case, the lever support part 123 is fixed so as not to be moved, and only the elastic lever 121 may be moved toward the lever support part 123. Accordingly, as described with reference to FIG. 6, the width between the roller support parts 111a and 111b may be increased (from the width W1 to the width W2).

FIG. 7 is a schematic view for describing a method for coupling the roller according to some exemplary embodiments.

Referring to FIG. 7, for example, the width between the roller support parts 111a and 111b may be increased by the pressure F applied to the elastic lever 121. Thereafter, the roller 150 may be inserted into a region between the roller support parts 111a and 111b.

For example, a rotation axis part 155b included in the inserted roller 150 may be coupled with a roller connection part 117b formed in a roller support part 111b. Thereafter, when removing the pressure F applied to the elastic lever 121, the elastic lever 121 is restored to its original state due to an elastic energy stored therein, and a roller connection part 117a may be coupled to a remaining rotation axis part 155a.

In some embodiments, referring to FIGS. 6 and 7, a second space 125 may be formed between the elastic lever 121 and the lever support part 123.

For example, the second space 125 may be a region where the elastic lever 121 is moved during applying the pressure F thereto. For example, as the elastic lever 121 is moved into the second space 125, tension may be applied to the roller support part 111a according to the lever principle.

In this case, the width between the roller support parts 111a and 111b may be more easily changed. Accordingly, the user may more easily couple or separate the roller 150 to or from the roller coupling part.

For example, a lever fixing part (not illustrated) may be inserted into the second space 125. In this case, it is possible to prevent a problem in which the roller 150 is arbitrarily separated during use of the device 100 for skin care, cosmetics and medical treatment.

## Claims

1. A device for skin care, cosmetics and medical treatment comprising:
a roller to which microneedles are coupled; and
a handle unit for movement coupled to the roller to move the roller, wherein the handle unit for movement comprises:
a roller coupling part to which the roller is detachably coupled;
an elastic pressing part connected to the roller coupling part to adjust a width thereof; and
a grip part extended from the elastic pressing part.

2. The device for skin care, cosmetics and medical treatment according to claim 1, wherein the roller is coupled with a microneedle sheet having the microneedles formed thereon.

3. The device for skin care, cosmetics and medical treatment according to claim 1, further comprising a cushioning cylinder mounted on the roller to cushion an impact applied to the roller.

4. The device for skin care, cosmetics and medical treatment according to claim 3, wherein the microneedles are coupled to the cushioning cylinder mounted on the roller.

5. The device for skin care, cosmetics and medical treatment according to claim 1, wherein the roller coupling part is deformed so that the roller is attached thereto or detached therefrom by a tension transmitted from the elastic pressing part due to a pressure applied thereto and converted into a tensile force.

6. The device for skin care, cosmetics and medical treatment according to claim 5, wherein the roller coupling part comprises a pair of roller support parts facing each other with the roller interposed therebetween.

7. The device for skin care, cosmetics and medical treatment according to claim 6, wherein a width between the roller support parts is changed by the tension.

8. The device for skin care, cosmetics and medical treatment according to claim 6, wherein the roller comprises rotation axis parts, and
the roller support parts comprises roller connection parts coupled with the rotation axis parts.

9. The device for skin care, cosmetics and medical treatment according to claim 6, wherein regions between the pair of roller support parts comprise:
a coupling region into which the roller is inserted; and
a first space excluding the coupling region among the regions between the roller support parts.

10. The device for skin care, cosmetics and medical treatment according to claim 1, wherein the elastic pressing part comprises: an elastic lever to which a pressure is applied; and a lever support part facing the elastic lever with being spaced apart therefrom.

11. The device for skin care, cosmetics and medical treatment according to claim 10, wherein a second space is formed between the elastic lever and the lever support part.

12. The device for skin care, cosmetics and medical treatment according to claim 10, wherein the grip part is extended from the lever support part.
